(19) Europäisches Patentamt
European Patent Office
Office européen des brevets

(11) EP 0 492 497 B1

(12) EUROPÄISCHE PATENTSCHRIFT

(45) Veröffentlichungstag und Bekanntmachung des Hinweises auf die Patenterteilung:
**28.08.1996 Patentblatt 1996/35**

(51) Int Cl.⁶: **C12P 7/62**, C12N 11/08

(21) Anmeldenummer: **91121933.5**

(22) Anmeldetag: **20.12.1991**

(54) **Verfahren zur Acylierung von Alkoholen mit einem immobilisierten Pseudomonas-Lipase**

Process for acylating alcohols with immobilized pseudomonaslipase

Procédé d'acylation d'alcools avec la lipase de pseudomonas immobilisée

(84) Benannte Vertragsstaaten:
**AT BE CH DE DK FR GB IT LI NL**

(30) Priorität: **24.12.1990 DE 4041777**

(43) Veröffentlichungstag der Anmeldung:
**01.07.1992 Patentblatt 1992/27**

(73) Patentinhaber: **HOECHST AKTIENGESELLSCHAFT**
**65926 Frankfurt am Main (DE)**

(72) Erfinder:
- **Schudok, Manfred, Dr.**
  **W-6230 Frankfurt am Main (DE)**
- **Fülling, Gerd, Dr.**
  **W-6230 Frankfurt am Main (DE)**
- **Kretzschmar, Gerdhard, Dr.**
  **W-6236 Eschborn 2 (DE)**

(56) Entgegenhaltungen:
**EP-A- 0 140 542**
**EP-A- 0 321 918**
**EP-A- 0 322 213**
**EP-A- 0 357 009**
**WO-A-89/01032**
**WO-A-90/04033**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 492 497 B1

## Beschreibung

Optisch aktive Alkohole sind oftmals wichtige chirale Vorstufen von biologisch aktiven Substanzen, wie z.B. von Arzneimitteln, Naturstoffen, Pflanzenschutzmitteln oder auch von Flüssigkristallbausteinen.

Ein wirtschaftliches Herstellungverfahren, welches die enzymatische Racematspaltung und damit die Darstellung der optisch aktiven Alkohole gewährleistet, ist deshalb von großer Bedeutung. Gleiches gilt für die enzymatische Stereodifferenzierung prochiraler Verbindungen, wie z.B. der Veresterung enantiotoper Hydroxylgruppen von 2-substituierten Propan-1,3-diolen. Außerdem ist die Acylierung mittels Enzymkatalyse, im Gegensatz zur chemischen Acylierung, für besonders empfindliche Substrate wie z.B. bestimmte primäre oder sekundäre Alkohole von Bedeutung.

Einige pharmakologische Wirkstoffe, deren Darstellung mit dem erfindungsgemäßen Verfahren erleichtert und wirtschaftlicher werden wird, sind Präparate wie NSAIDs (non-steroidal-antiinflammatory drugs), Betablocker, Bronchospasmolytica, Antimycotica, Pyrethroide, Tetramisol, Tetrahydrozolin, (R)-(-)-Toxometin und (S)-(+)-Fluoxetin sowie Prostaglandine und Kohlenhydrate. Chirale Bausteine zur Synthese von Protease-Inhibitoren, beispielsweise des Renins sind durch die Anwendung enzymatischer Verfahren erheblich einfacher zugänglich.

Es ist bereits bekannt, daß man Vinylester unter Hinzugabe von Alkoholen in Gegenwart von Lösungsmitteln, wie z.B. Tetrahydrofuran, unter enzymatischer Katalyse umestern kann (M. Degueil-Castaing et al. Tetrahedron Letters, Vol. 28, No. 9, Seiten 953-954, 1987). Als Enzym wurde Schweinepankreas-Lipase benutzt. Eine Stereoselektivität wurde nicht beobachtet.

In der WO 89/01032 wird die Verwendung von auf Polystyrolträgern immobilisierter Lipase aus Pseudomonas cepacia für enzymkatalysierte Umesterungsreaktionen vorgeschlagen.

Es ist außerdem die enzymatische Trennung von racemischen Alkoholen aufgrund einer selektiven enzymkatalysierten Umesterungsreaktion mit Vinylestern in Abwesenheit von Lösungsmitteln bekannt. Als Enzyme dienen immobilisierte Lipasen aus Schweineleber und -pankreas sowie aus den Mikroorganismen Pseudomonas, Candida, Mucor, Rhizopus und Penicillium (EP 032 19 18). In der EP O 321 918 wird ferner die Anregung gegeben, das Enzym in immobilisierter Form einzusetzen.

Weiterhin ist bekannt, daß für die Umesterung Carbonsäureester (G. Carpani, F. Orsini, M. Sisti, L. Verotta, Gazz. Chim. Ital. 119, p. 463-465 (1989)) bzw. für die Acylierung cyclische Carbonsäureanhydride (Y. Terao et al., Chem. Pharm. Bull. 37, p. 1653-1655 (1989)) eingesetzt werden können.

In der europäischen Patentanmeldung EP 0 25 42 43 werden aus prochiralen Diolen durch Umsetzung mit Vinylacetat in Gegenwart von Hydrolasen chirale Verbindungen optisch rein dargestellt. Dies gelingt durch selektive Veresterung nur einer der beiden enantiotopen primären OH-Gruppen.

Es ist außerdem bekannt, daß Lipasen, bei der Hydrolyse und Umesterung von Fetten, Ölen und ähnlichen Verbindungen immobilisiert eingesetzt werden können (M. Mittelbach. J. Am. Oil. Chem. Soc. 67, 168-170 (1990)).

Hsu et al. (Tetrahedron Letters, Vol. 31, No. 44, S. 6403-6406 (1990)) beschreiben die Umsetzung von sekundären Alkoholen mittels XAD-8 immobilisierter Lipase aus Pseudomonas und finden eine beschleunigte Umsetzung des Substrates. In der Publikation befinden sich jedoch keine Aussagen bezüglich der Standzeiten (Haltbarkeit) oder der Temperaturstabilität des fixierten Enzyms ohne nennenswerten Aktivitätsverlust.

In der EP 0 357 009 A2 wird zur enzymkatalysierten Racematspaltung von prochiralen oder chiralen Alkoholen durch Umesterung mit Enolestern die Verwendung von auf Polystyrolharzträgern immobilisierten Lipasen vorgeschlagen.

Es wurde nun überraschend gefunden, daß die O-Acylierung von Alkoholen mit Hilfe von immobilisierter Pseudomonas-Lipase besonders effektiv durchgeführt werden kann, indem man das Enzym mittels Bindung an hydrophobe Träger, wie Adsorberharze auf Polystyrolbasis mit definierten Porenvolumina, Oberflächen und Porendurchmessern immobilisiert.

Die Erfindung betrifft somit:

Ein Verfahren zur Acylierung von Alkoholen, wobei man einen Vinylester der Formel I

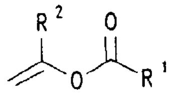

(I)

in der

$R^1$ Wasserstoff, $C_1$-$C_{18}$-Alkyl, welches gegebenenfalls mit Halogen substituiert ist, Phenyl oder ($C_1$-$C_3$)-Alkoxy-($C_1$-$C_4$)-Alkyl

und

$R^2$ Wasserstoff oder Methyl bedeutet,

in Gegenwart von immobilisierter Pseudomonas-Lipase mit einem Alkohol umsetzt,
das dadurch gekennzeichnet ist, daß die Pseudomonas-Lipase an einem Adsorberharz auf Polystyrolbasis mit einem Porenvolumen von 35 bis 55 %, einer Oberfläche von 200 bis 750 $m^2/g$, sowie einem Porendurchmesser von 50 bis 250 Å immobilisiert ist.

Im folgenden wird die Erfindung detailliert beschrieben, insbesondere in ihren bevorzugten Ausführungsformen.

Bei dem erfindungsgemäßen Verfahren wird der als Lösemittel dienende oder in einem anderen organischen Lösemittel gelöste Vinyl- bzw. Methylvinylester in ein Keton, Aldehyd oder Alkohol und einen Acylrest gespalten, und letzterer mit dem hinzugegebenen Alkohol (Substrat) enzymatisch acyliert.

Alle gemäß der vorliegenden Erfindung bevorzugten Trägermaterialien sind handelsüblich erhältlich.

Als Enzym werden Pseudomonas-Lipasen [Lipase P aus Pseudomonas cepacia (auch als FP oder PS bezeichnet) Amano Pharmaceuticals, Nagoya, Japan] eingesetzt.

Zur Immobilisierung des Enzyms werden pro 10 ml Träger 0,01 bis 2 g, bevorzugt jedoch 0,1-1,5 g Enzym in 0,005-1 M Kaliumphosphatpuffer, pH 5-9, bevorzugt jedoch pH 6-8, für 1-20 Std. gerührt. Der Puffer wird nach der Reaktionszeit über eine Fritte abgesaugt und das Enzym-Träger-Gemisch mit großen Mengen Wasser, Aceton und Vinylacetat gewaschen. Der Träger ist in diesem Zustand gebrauchsfertig und kann in trockenem Zustand gelagert werden.

Die mit Enzym zu belegende Trägermenge wird in Abhängigkeit von der Größe des Ansatzes, von der Reaktivität des Alkohols, von der zu erwartenden Reaktionszeit und von der gewünschten Umsatzhöhe frei gewählt. Sie kann durch Vorversuche leicht bestimmt werden.

Die Vinyl- bzw. Methylvinylester der Formel I sind, sofern nicht käuflich, auf einfache Weise herstellbar, beispielsweise durch Edelmetall-katalysierte Umesterung von Vinylacetat mit den entsprechenden Carbonsäuren. Vorzugsweise wird die Umesterung durch $Pd^{2+}$ katalysiert.

Die Vinylester können außerdem noch durch eine $Hg^{2+}$-katalysierte Addition von Acetylen synthetisiert werden.

Die Alkohole erhält man, sofern sie nicht käuflich sind, z.B. durch Reduktion aus den entsprechenden Ketonen, die meist käuflich sind, oder durch α-Halogenierung entsprechender Ketone mit anschließender Reduktion zum Alkohol. Andere nicht käufliche Alkohole oder Ketone lassen sich nach literaturbekannten Verfahren einfach herstellen, beispielsweise über Grignard- oder andere gängige Additionsreaktionen.

Unter Alkoholen versteht man einen Alkohol der Formel III

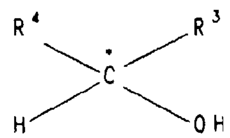

(III)

in der

$R^3$ $C_1$-$C_{18}$-Alkyl oder $C_3$-$C_{10}$-Cycloalkyl bedeutet, wobei diese Reste auch halogensubstituiert sein können,

und

$R^4$ Epoxy-$C_1$-$C_5$-Alkyl bedeutet, wobei die Epoxy-Gruppe in β-Position zur OH-Gruppe im Rest der Formel II steht

oder

$R^4$ $C_1$-$C_{10}$-Alkyl, $C_2$-$C_{10}$-Alkenyl, $C_2$-$C_{10}$-Alkinyl, $C_3$-$C_8$-Cycloalkenyl, wobei die Alkyl-, Alkenyl-, Alkinyl- und Cycloalkenylreste gegebenenfalls mit COOH, Halogen, $NO_2$, CN, $C_1$-$C_4$-Alkoxycarbonyl oder Phenyl substituiert sind, wobei der Phenylrest seinerseits mit Halogen, $NO_2$, CN oder $C_1$-$C_4$-Alkoxy substituiert sein kann, oder $R^4$ Aryl oder Heteroaryl bedeutet, wobei die Aryl- oder Heteroarylreste gegebenenfalls mit $C_1$-$C_4$-Alkyl, $C_1$-$C_4$-Alkoxy, Halogen, $NO_2$, CN oder N Sgr. substituiert sind, wobei Sgr eine Aminoschutzgruppe darstellt,

oder in der

$R^3$ und $R^4$ zusammen eine Alkylen- oder Alkenylen-Rest der Formel IVa, b

a) b)

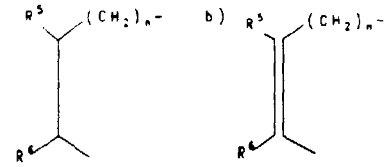

(IV)

darstellen, in der

n 1, 2 oder 3 ist und

$R^5$ und $R^6$ gleich oder verschieden sind und Wasserstoff, $C_2$-$C_4$-Alkenyl, $C_1$-$C_4$-Alkyl oder

$R^5$ und $R^6$ zusammen anelliertes Phenyl oder anelliertes Naphthyl bedeuten, wobei der Phenyl- oder Naphthyl-Rest gegebenenfalls $C_1$-$C_4$-Alkyl-, $C_1$-$C_4$-Alkoxy, $NO_2$-, CN- oder Halogen-substituiert ist,

wobei eine Methyleneinheit der Alkenylenkette auch durch eine Carbonylgruppe ersetzt sein kann,
oder
einen Alkohol der Formel V

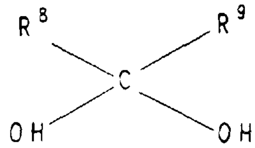

(V)

in der

$R^8$ Wasserstoff oder eine Alkylgruppe und
$R^9$ Alkyl, Aralkyl, Aryl, Benzyl oder eine Naphthylmethylgruppe

bedeuten.

Es können außerdem alle höherwertigen Alkohole als Substrat eingestzt werden.

Unter Halogenen des Alkohols der Formel III werden Fluor, Chlor, Brom und Jod, insbesondere Chlor und Brom verstanden. Unter "Aryl" werden beispielsweise Phenyl, Naphthyl, Phenanthryl, Anthryl und Fluorenyl verstanden, insbesondere Phenyl, Naphthyl und Phenanthryl. Unter "Heteroaryl" werden beispielsweise Furyl, Thienyl, Pyrrolyl, Pyridyl, Pyrimidyl, Pyrazinyl, Pyridazinyl, Pyrazolyl, Isoxazolyl, Thiophenyl, Imidazolyl, Oxazolyl, Thiazolyl und Indolyl verstanden, insbesondere Furyl, Thienyl, Pyrrolyl und Pyridyl. Unter der Aminoschutzgruppe "Sgr." werden die in der Peptidchemie üblicherweise eingesetzten Aminoschutzgruppen verstanden, beispielsweise Benzyloxycarbonyl (Z), Benzoyl, Benzyl, Butyloxycarbonyl (Boc), 9-Fluorenyl-methoxycarbonyl (Fmoc), Benzhydryl, Allyloxycarbonyl (Aloc), Tosyl, Methoxymethyl (MOM), Tetrahydropyranyl (THP), Acetyl, aber auch Alkyl- oder Cycloalkylgruppen, wie beispielsweise N-Methyl oder N,N-Dimethyl. Unter "anelliertem Phenyl" bzw. "anelliertem Naphthyl" wird ein Phenyl- bzw. Naphthylrest verstanden, bei dem die C-C-Doppelbindung des Restes der Formel III Bestandteil des Phenyl- bzw. Naphthylrestes ist. Die gegebenenfalls substituierten Reste $R^1$, $R^3$, $R^4$, $R^5$ und $R^6$ sind bevorzugt monosubstituiert.

Alkyl- und Alkenylreste mit 3 und mehr Kohlenstoffatome und Alkinylreste mit 4 und mehr Kohlenstoffatomen können sowohl geradkettig als auch verzweigt sein.

Der zu acylierende Alkohol wird in Konzentration von 0,05-200 %, vorzugsweise 0,5-10 %ig - bezogen auf das Volumen des Vinylesters - eingesetzt.

Für die Acylierung des Alkohols sind mindestens 0,5 Molärquivalente des Vinylrestes einzusetzen.

Die Umsetzung der Alkohole erfolgt "batchweise" oder im kontinuierlichen Verfahren.

Die Racematspaltung der Alkohole kann beim erfindungsgemäßen Verfahren mit einer Aktivitätserhöhung von mindestens 90 % verglichen mit herkömmlichen Verfahren durchgeführt werden.

Das trägerfixierte Enzym zeigt im kontinuierlichen Verfahren selbst nach einigen Monaten der Verwendung, wobei sich Reaktionsläufe und Phasen der Nichtbenutzung des fixierten Enzyms abwechseln, kaum Aktivitätsverlust. Dies gilt auch, wenn die Reaktion bei erhöhten Temperaturen durchgeführt wird.

Für die "batchweise"-Umsetzung mit immobilisierter Pseudomonas-Lipase wird der Vinylester der Formel I, vorzugsweise jedoch Vinylacetat oder eine Lösung des Vinylesters, in einem (unpolarem) organischen Lösemittel vorgelegt und der umzusetzende Alkohol hinzugegeben. Als Lösemittel eignen sich vorzugsweise Ether, ganz besonders jedoch symmetrische und unsymmetrische, verzweigte und nicht verzweigte Dialkylether. Außerdem eignen sich vorzugsweise Kohlenwasserstoffe, ganz besonders lineare, verzweigte oder cyclische Kohlenwasserstoffe von $C_4$-$C_8$. Zu dieser Suspension gibt man trägerfixierte Pseudomonas-Lipase und rührt oder schüttelt bei konstanter Temperatur. Die Beendigung der Reaktion wird mittels DC, GC oder HPLC kontrolliert. Anschließend filtriert man das fixierte Enzym ab, wäscht gut mit einem Lösemittel (s.o.) oder Vinylacetat nach und dampft die Lösung im Vakuum ein. Das im Fall der Racemattrennung als Rückstand verbleibende Alkohol-/Ester-Gemisch wird durch Säulenchromatographie an Kieselgel oder durch Extraktion, Kristallisation oder Destillation getrennt. Andere Acylierungsprodukte fallen häufig in ausreichender Reinheit an, so daß sich eine Aufreinigung erübrigt.

Zur Durchführung der Reaktion im kontinuierlichen Verfahren packt man die trägerfixierte Pseudomonas-Lipase P/FP/PS in eine Glassäule und spült mit dem Lösemittel, in dem die Reaktion durchgeführt wird, d.h. mit Vinylacetat, einem anderen Vinylester oder einem anderen organischen Lösemittel.

Anschließend läßt man die Substratlösung mit konstanter Geschwindigkeit bei konstanter Temperatur durchlaufen.

Das Verhältnis von Träger [ml] zu fixierter Lipase [g] zu Vinylacetat [ml] zur Konzentration des Substrats [Vol-%] liegt im Bereich 5-100:1:5-10.000:0,5-200, wenn Vinylacetat (d.h. ohne weiteren Lösemittelzusatz) eingesetzt wird.

Wird die Reaktion in einem Vinylester durchgeführt, setzt man den zu acylierenden Alkohol in Konzentrationen von 0,05 - 200 Vol-% ein.

Die Höhe des Umsatzes kann nahezu beliebig durch Einstellen der Tropfgeschwindigkeit gesteuert werden und ist durch Vorversuche leicht zu bestimmen.

Die Raum-Zeit-Ausbeuten sind direkt von den Absolutwerten der oben genannten Parametern abhängig, besonders aber von der Säulendimensionen, d.h. der Enzymmenge, die trägerfixiert in der Säule vorgelegt wird.

Die Säulendimension ist frei wählbar, liegt im Labormaßstab jedoch vorzugsweise in einer Größenordnung von 10-500 ml. Bei einer bevorzugten Säulenbefüllung mit 50 ml trägerfixiertem Enzym, einer 1 %igen Substratlösung und einer Durchlaufgeschwindigkeit von 10 Tropfen/Min. werden Raum-Zeit-Ausbeuten von etwa 0,5-300 g/l/h erzielt.

Die Reaktionstempertur während des Verfahrens beträgt (-) 10 bis (+) 100°C, vorzugsweise (+) 0-60°C.

Die Reaktionszeiten sind abhängig von der Art des umzusetzenden Alkohols, dessen Konzentration sowie der Menge des trägerfixierten Enzyms und schwanken zwischen 1 Std. und 4 Wochen. Vorzugsweise liegen sie zwischen 3 Std. und 3 Tagen.

Die bei dem erfindungsgemäßen Verfahren entstehenden Produkte Acetaldehyd bzw. Aceton bzw. die für die Acylierung freigesetzten Alkohole sowie die im Falle der selektiven Acylierung entstehenden enantiomeren Alkohole (Substrate), d.h. Carbonsäureester und nicht umgesetzter Alkohol lassen sich auf bekannte Art und Weise durch Anwendung aller, aber im Einzelfall zu prüfender, üblicher Methoden trennen, vorzugsweise durch Chromatographie an Kieselgel oder eines der anderen, oben erwähnten Verfahren.

Beispiel

Allgemeine Arbeitsvorschrift

Zur Immobilisierung des Enzyms wird das Trägermaterial entweder

a) unbehandelt eingesetzt,
b) nach der Enzymimmobilisierung mit Glutardialdehyd nachvernetzt (Tab. 1)

zu a) Für die Fixierung der Lipase am Träger werden 50 ml des Trägers in 100 ml K-Phosphatpuffer, pH 7,0, suspendiert und mit 500 mg Lipase P versetzt. Man rührt für 3 h bei RT, filtriert ab und wäscht gut mit Wasser nach.

zu b) Nachvernetzung
Zur Nachvernetzung wird wie unter a) beschrieben verfahren, jedoch nach der angegebenen Fixierungszeit mit 4 ml Glutardialdehydlösung (25 %ig) vernetzt. Nach 1 Stunde wird das trägerfixierte Enzym abfiltriert und mit Wasser nachgewaschen.

Die bevorzugten Träger können wie folgt charakterisiert werden:

| | ®XAD-2 | ®XAD-4 |
|---|---|---|
| Porenvolumen [%] | 42 | 51 |
| Dichte | 1,02 | 1,02 |
| Oberfläche [$m^2$/g] | 330 | 750 |

(fortgesetzt)

| | ®XAD-2 | ®XAD-4 |
|---|---|---|
| Porendurchmesser [Å] | 90 | 50 |

Der Träger wird in Wasser oder trocken aufbewahrt.

500 mg des umzusetzenden Alkohols werden in 20 ml Vinylacetat suspendiert. Dazu gibt man die immobilisierte Lipase und rührt bei konstanter Temperatur.

Nach Beendigung der Reaktion filtriert man das immobilisierte Enzym ab. Die verbleibende Lösung wird im Vakuum vollständig eingeengt.

Die im Rückstand befindlichen Acylierungsprodukte können durch Standardverfahren, beispielsweise durch Kieselgelchromatographie, getrennt werden.

In Tabelle 2 sind die Ausgangs- sowie die erhaltenen Produkte, die variablen Verfahrensparameter (Enzymmenge, Trägermenge, Alkoholmenge, Vinylestermengen, Reaktionstemperatur, Reaktionszeit) sowie Produktcharakteristika und chemische Ausbeute angegeben.

Zur exakten Bestimmung der Aktivität ist es notwendig, die an den Träger gebundene Enzymmenge genau zu bestimmen.

Man geht bei den hier dargestellten Versuchen für das erfindungsgemäße Verfahren von der Voraussetzung aus, daß Pseudomonas-Lipase ein einheitliches Protein mit einem Salzgehalt von 37 % bzw. 63 % Protein (Gewichts-%) ist. Dieser Salzgehalt wird durch Dialyse bestimmt.

Für die Bestimmung der Fixierungsausbeute macht man den schon beschriebenen Versuch mit einem Ansatz von 20 ml XAD-2 Träger und 2 g Pseudomonas-Lipase. Die Fixierungs- und Waschlösung werden gesammelt, vereinigt und lyophilisiert. Aus diesem Pool erhält man 1,77 g Rückstände, bestehend aus Enzym und Puffersalzen. Durch Auswaage nach dem Lyophilisieren von reinem Puffer ist eine Salzmenge von 0,38 g in der Menge der eingesetzten Pufferlösung bekannt.

Somit sind von den 1,77 g Rückstand zum einen 0,38 g Puffersalze und zum anderen 0,74 g Salze des Enzyms (=37 %; s.o.) abzuziehen.

Die verbleibende Menge von 0,65 g sollte der nicht fixierten Enzymmenge entsprechen.

Eine nachfolgende Dialyse des Rückstandes zeigt, daß noch eine geringe Salzmenge enthalten ist, so daß 0,58 g Enzymmenge statt des theoretischen Wertes von 0,65 g verbleiben.
Somit liegt die fixierte Enzymmenge zwischen 0,61 und 0,68 g. Daraus folgt, daß die Fixierungsausbeute 51 % beträgt.

Die Berechnung wird nochmals beispielhaft für das in Tabelle 2 genannte Beispiel 1 (Phenylethanol) durchgeführt.

50 mg Lipase P (käuflich erworben)

x 0,63 tatsächlicher Enzymgehalt Lipase P

x 0,51 Fixierungsausbeute

$\Rightarrow$ 16 mg trägerfixierte Lipase

50 mg freie Lipase P

x 0,63 tatsächlicher Enzymgehalt Lipase P

$\Rightarrow$ 31,5 mg Lipase P tatsächlicher Enzymgehalt

16 mg fixiertes Enzym liefern 33,4 % Umsatz

31,5 mg freies Enzym liefern 20,7 % Umsatz

$\Rightarrow$ Aktivität: 320 %

Die enzymatische Racematspaltung kann nicht nur, wie oben beschrieben, in Anwesenheit von Vinylacetat, sondern auch in Anwesenheit anderer Vinylester, wie z. B. von Chloressigsäure, Laurinsäure und Phenylessigsäure erfolgen. Hierfür wird Träger-fixierte Lipase P in eine Glassäule gefüllt und mit 150 ml t-Butylmethylether, der als Lösemittel für die folgende Umsetzung dient, durchspült.

Anschließend beschickt man die Säule mit einer Lösung von Substrat und Vinylester, die in jeweils 250 ml t-Butylmethylether gelöst sind. Diese Lösung läßt man die Säule langsam durchlaufen und bestimmt die Zusammensetzung nach vollständiger Passage der Lösungen mittels Gaschromatographie.

Die einzusetzenden Mengen, sowie die Durchlauf- und Reaktionszeiten und die Ergebnisse sind Tabelle 3 zu entnehmen.

Beispiel für die Umesterung mit Essigsäureethylester:

0,5 ml Essigsäureethylester werden mit 2,5 %igem Phenylethanol in t-Butylmethylether (10 ml) und 5 ml XAD-2 fixiertem Enzym (theoret. Enzymmenge: 50 mg) bei Raumtemperatur gerührt.
Die Reaktion zeigte einen 50 %igen Umsatz des Phenylethanols nach 5 Tagen.

Die Kontrolle mit nicht fixiertem Enzym ergab nach 7 Tagen einen deutlich geringeren Umsatz.

Der Umsatz wurde mittels DC bestimmt.

Beispiel für die Veresterung primärer OH-Gruppen:

2,5 %iges Geraniol werden in 10 ml Vinylacetat gelöst und unter Zusatz von 5 ml XAD-2 fixiertem Enzym (theoret. Enzymmenge: 50 mg) bei Raumtemperatur gerührt.
Eine quantitative Acetylierung war mit fixiertem Enzym bereits nach 1 Std. durchgeführt, während das freie Enzym 50 % mehr Zeit benötigte.

Beispiel für die Acetylierung von dl-Pantolacton - Test der Langzeitstabilität bei erhöhter Temperatur im kontinuierlichen Verfahren:

400 ml XAD-2-fixierte Lipase P wird in eine temperierbare Doppelmantel-Glassäule gepackt. Eine 0,1 %ige Lösung von dl-Pantolacton (in Vinylacetat/t-Butylmethylether 1:9; Gesamtvolumen 2 l) wird bei 50° C mit einer Durchflußgeschwindigkeit von 0,11 ml/min über die Säule gegeben. Zur Kompensation des Säulenvolumens wird anschließend mit gleicher Geschwindigkeit mit t-Butylmethylether nachgespült. GC-Analyse der Reaktionslösung ergibt in diesem ersten Durchlauf einen Umsatz zu Pantolactonacetat von 71,6 %. Die gespülte Säule wird bei RT im trockenen Zustand aufbewahrt und jeweils 5 h vor erneutem Beginn eines weiteren Durchlaufs temperiert. Auf diese Art und Weise werden innerhalb der nächsten 5 Monate noch 11 weitere kontinuierliche Umsetzungen durchgeführt. Der 12. Lauf (unter identischen Bedingungen, außer: Durchflußgeschwindigkeit 0,13 ml/min), 5 Monate später durchgeführt, zeigt einen Umsatz zu Pantolactonacetat von 70,7 %.

Tabelle 1:

| Die Reaktionszeit beträgt jeweils 6 Std. | | | | | | | | |
|---|---|---|---|---|---|---|---|---|
| Bsp. Nr. | Träger | mit Chloressigsäure vorfunkt. | mit Glutardialdehyd nachvernetzt | % Umsatz zu Acetat | | | | Einsatzmenge |
| | | | | 1. Lauf | 2. | 3. | 4. | |
| | | | | | | | | |
| 1 | ®Amberlite XAD-2 | - | - | 33,4 | 38,4 | 38 | 33,5 | (X) |
| 2 | XAD-4<br>XAD-16<br>XAD-1180 | -<br>-<br>- | -<br>-<br>- | 19,35<br>17,20<br>17,61 | 11,94<br>14,11<br>13,03 | 12,31<br>14,91<br>12,71 | 8,08<br>10,06<br>11,19 | |
| 3 | XAD-12 | - | - | 19 | 4,45 | - | - | |
| 4 | XAD-2 | - | ja | 18,45 | 24,93 | 17,31 | 13,46 | |
| 5 | XAD-4 | - | ja | 15,66 | 16,74 | 14,63 | 11,91 | |
| 6 | XAD-12 | - | ja | 26,06 | 9,7 | - | - | |
| 7 | ®DEAE-Sephadex | - | - | 11,03 | - | - | - | dto. |

Tabelle 1: (continued)

| Die Reaktionszeit beträgt jeweils 6 Std. | | | | | | | | |
|---|---|---|---|---|---|---|---|---|
| Bsp. Nr. | Träger | mit Chloressigsäure vorfunkt. | mit Glutardialdehyd nachvernetzt | % Umsatz zu Acetat | | | | Einsatzmenge |
| | | | | 1. Lauf | 2. | 3. | 4. | |
| 8 | ®DEAE-Sephadex | - | ja | 12,58 | - | - | - | |
| 9 | /Sephacel | - | - | 7,03 | - | - | - | |
| 10 | /Sephacel | - | ja | 8,55 | - | - | - | |
| 11 | ®Celite | - | - | 10,98 | - | - | - | |
| 12 | ®Duolite A7 | - | ja | 25,96 | 12,7 | 12,65 | - | |
| 13 | ®"A 368" | - | ja | 41,13 | 5,61 | 5,5 | - | |
| (x) In Bsp. 1 wurden verwendet: 250 mg Phenylethanol, 10 ml Vinylacetat, 5 ml Träger | | | | | | | | |

Tabelle 2 (Als Träger wurde jeweils Amberlite XAD-2 verwendet)

| Bsp. Nr. | racemo: prochir. Alkohol | Edukt Menge [g] | Konz. in % | Menge A: Träger [ml] | Menge B: Enzym [g] | Temp. [°C] | Menge Vinylester [ml] | Reaktionszeit [h] | Umsatz [%] | Akt [%] |
|---|---|---|---|---|---|---|---|---|---|---|
| 1 | 1-Phenyl-ethanol | A: 0,25 | 2,5 | 5 | 0,05 | RT | 10 | 6 | 33,4 | 320 |
| | | B: 0,25 | 2,5 | | 0,05 | RT | 10 | 6 | 20,7 | - |
| | | C: 0,5 | 1 | 50 | 5 | RT | 50 | - | 50 | - |
| 2 | 1-Phenyl-propanol | A: 0,50 | 2,5 | 10 | 1 | RT | 20 | 30 | 46,1 | 250 |
| | | B: 0,52 | 2,5 | | 1 | RT | 20 | 30 | 37,1 | - |
| | | C: - | - | - | - | RT | - | - | - | - |
| 3 | 2-Chlor-1-phenylethanol | A: 0,51 | 2,5 | 10 | 1 | RT | 20 | 48 | 51,7 | 320 |
| | | B: 0,51 | 2,5 | | 1 | RT | 20 | 48 | 32,3 | - |
| | | C: 0,5 | 0,2 | 50 | 5 | RT | 50 | - | 34 | - |
| 4 | Pantolacton | A: 0,5 | 2,5 | 10 | 1 | RT | 20 | 48 | 32,2 | 220 |
| | | B: 0,49 | 2,5 | | 1 | RT | 20 | 48 | 29,7 | - |
| | | C: 0,5 | 0,2 | 50 | 5 | 50°C | 250 | - | 42 | - |
| 5 | Allethrolon | A: 0,5 | 2,5 | 10 | 1 | RT | 20 | 4 | 45 | 210 |
| | | B: 0,51 | 2,5 | | 1 | RT | 20 | 4 | 42 | - |
| | | C: 0,5 | 1 | 50 | 5 | RT | 500 | - | 64 | - |

Fortsetzung Tabelle 2

| Bsp. Nr. | racemo: prochir. Alkohol | R-Z-Ausbeute [g/1/h] | Alkohol ee | Alkohol $\alpha_D^{25}$ | Alkohol chem. Ausbeute | Acetat ee | Acetat $\alpha_D^{25}$ | Acetat chem. Ausbeute |
|---|---|---|---|---|---|---|---|---|
| 1 | 1-Phenyl-ethanol | - | n. b. | n. b. | n. b. | > 95 % | +105,6 | n. b. |
| | | - | " | " | " | > 95 % | +105,1 | n. b. |
| | | 3 | > 95 % | -44 | 43 % | > 95 % | 104,1 | 44 % |
| 2 | 1-Phenyl-propanol | - | 77 % | -38 | 45 % | > 95 % | 102,7 | 36 % |
| | | - | 55 % | -27,4 | 54 % | > 95 % | +100,1 | 33 % |
| | | - | - | - | - | - | - | - |
| 3 | 2-Chlor-1 phenylethanol | - | n. b. | n. b. | 35 % | > 95 % | + 75,6 | 39 % |
| | | - | n. b. | n. b. | 62 % | > 95 % | - | 25 % |
| | | 0,5 | n. b. | n. b. | n. b. | n. b. | n. b. | n. b. |
| 4 | Pantolacton | - | n. b. | n. b. | 38 % | > 95 % | + 12,7 | 22 % |
| | | - | n. b. | n. b. | 63 % | > 95 % | + 12,7 | 28 % |
| | | 0,5 | n. b. | n. b. | n. b. | > 95 % | + 11,4 | 37 % |
| 5 | Allethrolon | - | > 95 % | +14,5 | 42 % | > 84 % | - 27,6 | 44 % |
| | | - | > 95 % | +14,8 | 43 % | 92 % | - 29,9 | 41 % |
| | | 6 | > 95 % | +14,6 | 28 % | 52 % | - 15,87 | 63 % |

Fortsetzung Tabelle 2

| Bsp. Nr. | racemo: prochir. Alkohol | Edukt Menge [g] | Konz. in % | Menge A: Träger [ml] | Menge B: Enzym [g] | Temp. [°C] | Menge Vinylester [ml] | Reaktionszeit [h] | Umsatz [%] | Akt. [%] |
|---|---|---|---|---|---|---|---|---|---|---|
| 6 | 1- (6-Acetoxy) | A: - | - | - | - | | - | - | - | - |
| | napthyl- | B: - | - | - | - | | - | - | - | - |
| | ethanol | C: 14 | 1 | 50 | 5 | RT | 1400 | - | 52 | - |
| 7 | 2-(1-Naphthyl- | A: 1 | 2 | 2,5 | 0,25 | 0°C | 50* | 16 | 98 | 190 |
| | methyl)-prop- | B: 1 | 2 | | 0,25 | 0°C | 50* | 16 | 95 | - |
| | an-1,3-diol | C: 1 | 0,5 | 50 | 5 | 15°C | 200* | 6 | n. b. | - |

A = "Batch"-Versuch mit immob. Enzym

B = Vergleichsansatz mit freiem Enzym

C = Versuch im kontinuierlichen Verfahren

RT = Raumtemperatur

n. b. = nicht bestimmt

* verwendet wurde Gemisch Vinylacetat/Dimethoxyethan/Diethylether

| Bsp. Nr. | racemo: prochir. Alkohol | R-Z-Ausbeute [g/l/h] | Alkohol ee | Alkohol $\alpha_D^{25}$ | Alkohol chem. Ausbeute | Acetat ee | Acetat $\alpha_D^{25}$ | Acetat chem. Ausbeute |
|---|---|---|---|---|---|---|---|---|
| 6 | 1- (6-Acetoxy) | - | - | - | - | - | - | - |
| | naphthyl- | - | - | - | - | - | - | - |
| | ethanol | 3 | > 95 % | -30 | 45 % | 92 % | + 82,35 | 48 % |
| 7 | 2-(1-Naphthyl- | - | - | - | - | 95 % | + 39,5 | 94 % |
| | methyl)-prop- | - | - | - | - | 97 % | + 40,3 | 91 % |
| | an-1,3-diol | 30 | - | - | - | 88 % | + 36,5 | n. b. |

ee: gemessen nach Drehwert

Tabelle 3: Acylierung von Phenylethanol in unterschiedlichen Vinylestern

| Bsp. Nr. | rac. + Alkohol [g] | Vinylester Art / Menge [ml] | Menge Enzym/Träger* | Reaktionszeit [h] | Umsatz (%) |
|---|---|---|---|---|---|
| 1 | 2,5 g | Chloressigsäure / 12,5 | 5/50 | 5 | 67,6 |
| 2 | 2,5 g | Laurinsäure / 5 | 5/50 | 5 | 41,7 |
| 3 | 2,5 g | Phenylessigsäure / 5 | 5/50 | 16 | 24,4 |

+ Als rac. Alkohol wurde jeweils Phenylethanol eingesetzt.
* Als Träger wurde jeweils ®Amberlite XAD-2 eingesetzt.

Das erfindungsgemäße Verfahren weist gegenüber herkömmlichen Verfahren zur Racematspaltung von Alkoholen folgende Vorteile auf:

A) Durch erhöhte Enzymaktivität sind die Raum-Zeit-Ausbeuten deutlich erhöht.

B) Sehr hohe Standzeiten des fixierten Enzyms erlauben einen besonders wirtschaftlichen Einsatz des Biokatalysators.

C) Die Aktivität des Enzyms ist dauerhaft (Tab. 1; s.u. (a)).

D) Hohe Temperaturstabilität des fixierten Enzyms. 10 ml 0,5 %ige Phenylethanollösung in Toluol wird mit 100 mg freier Lipase bzw. 1 ml fixiertem Enzym und anschließend mit jeweils 0,1 ml Phenylessigsäurevinylester vesetzt. Die freie Lipase zeigt nach fünfstündigem Rühren bei RT 3,2 % Umsatz bzw. nach gleichem Zeitablauf unter Rückfluß mit 110° C keine Aktivität mehr, während die fixierte Lipase bei 110° C noch 1-2 % Umsatz aufweist. Die Bestimmung des Umsatzes erfolgt per GC Test (Reoplex auf ®Chromosorb).

(a) 250 mg racem. Phenylethanol in 20 ml Vinylacetat wird mit 2 ml fixierten Lipase bei 50° C für 6 h geschüttelt. Anschließend wird der Umsatz per GC bestimmt, das fixierte Enzym abfiltriert und gut mit Vinylacetat nachgewaschen. Am nächsten Tag erfolgt eine erneute Umsetzung unter identischen Bedingungen.
(b) In gleicher Weise wird unter Verwendung von 0,2 g des freien Enzyms verfahren.
(a) Im 1. Durchlauf wird nach 6 h ein Umsatz von 48,7 % gemessen. Im 10. Durchlauf wird nach 6 h ein Umsatz von 21,2 % gemessen.
(b) Im ersten Durchlauf liegt der Umsatz nach 6 h bei 46,1 %. Nach dem 7. Durchlauf kein Produkt mehr nachweisbar.

Die Aktivität des Enzyms ist auf Dauer gewährleistet.

**Patentansprüche**

1. Verfahren zur Acylierung von Alkoholen, wobei man einen Vinylester der Formel I,

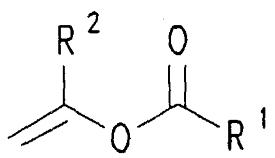

(I)

in der

$R^1$ Wasserstoff, $C_1$-$C_{18}$-Alkyl, welches gegebenenfalls mit Halogen substituiert ist, Phenyl oder $C_1$-$C_3$-Alkoxy-$C_1$-$C_4$-Alkyl

und

$R^2$ Wasserstoff oder Methyl bedeutet,
in Gegenwart von immobilisierter Pseudomonas-Lipase mit einem Alkohol umsetzt, dadurch gekennzeichnet, daß die Pseudomonas-Lipase an einem Adsorberharz auf Polystyrolbasis mit einem Porenvolumen von 35 bis 55 %, einer Oberfläche von 200 bis 750 $m^2/g$ und einem Porendurchmesser von 50 bis 250 Å immobilisiert ist.

2. Verfahren nach Anspruch 1, dadurch gekennzeichnet, daß die Umsetzung "batchweise" erfolgt.

3. Verfahren nach Anspruch 1, dadurch gekennzeichnet, daß die Umsetzung im kontinuierlichen Verfahren erfolgt.

4. Verfahren nach einem der Ansprüche 1 bis 3, dadurch gekennzeichnet, daß der Träger jeweils

a) unbehandelt zur Immobilisierung oder
b) nach Enzymankupplung mit Glutardialdehyd nachvernetzt, eingesetzt werden kann.

5. Verfahren nach einem der Ansprüche 1 bis 4, dadurch gekennzeichnet, daß die Reaktionstemperatur (-) 10 bis (+) 100° C beträgt.

6. Verfahren nach einem der Ansprüche 1 bis 5, dadurch gekennzeichnet, daß die Reaktionstemperatur 0 bis (+) 60° C beträgt.

**Claims**

1. A process for the acylation of alcohols, where a vinyl ester of the formula I

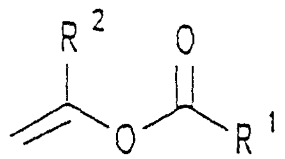

(I)

in which

$R^1$ is hydrogen, $C_1$-$C_{18}$-alkyl which is optionally substituted by halogen, or is phenyl or $C_1$-$C_3$-alkoxy-$C_1$-$C_4$-alkyl

and

$R^2$ is hydrogen or methyl,
is reacted with an alcohol in the presence of immobilized Pseudomonas lipase, wherein the Pseudomonas lipase is immobilized on a polystyrene-based adsorber resin with a pore volume of 35 to 55%, a surface area of 200 to 750 $m^2/g$ and a pore diameter of 50 to 250 Å.

2. The process as claimed in claim 1, wherein the reaction is carried out batchwise.

3. The process as claimed in claim 1, wherein the reaction is carried out in a continuous process.

4. The process as claimed in any one of claims 1 to 3, wherein the carrier can be employed in each case

   a) untreated for the immobilization or
   b) subsequently crosslinked with glutaraldehyde after coupling on of the enzyme.

5. The process as claimed in any one of claims 1 to 4, wherein the reaction temperature is (-)10 to (+)100°C.

6. The process as claimed in any one of claims 1 to 5, wherein the reaction temperature is 0 to (+)60°C.

**Revendications**

1. Procédé pour l'acylation d'alcools, dans lequel on fait réagir avec un alcool un ester vinylique de formule I

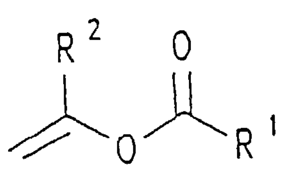

(I)

dans laquelle

$R^1$ représente un atome d'hydrogène ou un groupe alkyle en $C_1$-$C_{18}$ qui est éventuellement substitué par un halogène, le groupe phényle ou un groupe alcoxy($C_1$-$C_3$)-alkyle-($C_1$-$C_4$), et
$R^2$ représente un atome d'hydrogène ou le groupe méthyle, en présence d'une lipase immobilisée de *Pseudomonas,* caractérisé en ce que la lipase de *Pseudomonas* est immobilisée sur une résine adsorbante à base de polystyrène ayant un volume de pores de 35 à 55 %, une superficie de 200 à 750 $m^2/g$, ainsi qu'un diamètre de pores de 50 à 250 Å.

2. Procédé selon la revendication 1, caractérisé en ce que la réaction s'effectue en mode discontinu.

3. Procédé selon la revendication 1, caractérisé en ce que la réaction s'effectue dans le procédé en continu.

4. Procédé selon l'une des revendications 1 à 3, caractérisé en ce que le support peut être utilisé dans chaque cas

   a) non traité pour l'immobilisation ou
   b) post-réticulé avec du glutaraldéhyde après le couplage de l'enzyme.

5. Procédé selon l'une des revendications 1 à 4, caractérisé en ce que la température de la réaction est dans la plage allant de -10 à +100°C.

6. Procédé selon l'une des revendications 1 à 5, caractérisé en ce que la température de la réaction est dans la plage allant de 0 à 60°C.